# EUROPEAN PATENT SPECIFICATION

(11) **EP 1 541 163 B1**
(45) Date of publication and mention of the grant of the patent: **10.12.2008**
(21) Application number: 03791321.7
(22) Date of filing: 27.08.2003
(51) Int. Cl.: A61K 36/53, A61P 17/00, A61P 17/14

(54) **HAIR GROWTH STIMULANTS FOR ORAL USE**
MITTEL ZUR STIMULIERUNG DES HAARWACHSTUMS ZUR ORALEN ANWENDUNG
STIMULANTS DE CROISSANCE CAPILLAIRE ADMINISTRES PAR VOIE ORALE

(30) Priority: 28.08.2002 JP 2002248579
(43) Date of publication of application: 15.06.2005
(73) Proprietor: SUNSTAR INC., Takatsuki-shi, Osaka 569-1195 (JP); Nippon Shinyaku Co., Ltd., Kyoto-shi, Kyoto 601-8550 (JP)
(72) Inventor: NAKAOJI, Kouichi c/o SUNSTAR INC., Takatsuki-shi, Osaka 569-1195 (JP); MATSUURA, Masahiro c/o SUNSTAR INC., Takatsuki-shi, Osaka 569-1195 (JP); HAYASHI, Naoko c/o SUNSTAR INC., Takatsuki-shi, Osaka 569-1195 (JP); NISHI, Toyoyuki c/o NIPPON SHINYAKU CO., LTD., Kyoto-shi, Kyoto 601-8550 (JP)
(74) Representative: Müller-Boré & Partner Patentanwälte
(86) International application number: PCT/JP2003/010820
(87) International publication number: WO 2004/019962

(56) References cited:
- JP-A- 5 139 938
- JP-A- 7 002 677
- JP-A- 59 116 211
- JP-A- 2000 103 718
- JP-A- 2000 201 650
- JP-A- 2000 302 678
- JP-A- 2001 031 528
- JP-A- 2001 220 320
- JP-A- 2003 026 594
- JP-A- 2003 221 315
- MARIAM A ET AL: "Hypoglycaemic activity of the aqueous extract of Orthosiphon stamineus" 1996, FITOTERAPIA, VOL. 67, NR. 5, PAGE(S) 465-468 , XP009070103 ISSN: 0367-326X * the whole document *
- CASADEBAIG-LAFON J ET AL: "ELABORATION D'EXTRAITS VEGETAUX ADSORBES, REALISATION D'EXTRAITS SECS D'ORTHOSIPHON STAMINEUS BENTH" PHARMACEUTICA ACTA HELVETIAE, vol. 64, no. 8, 1989, pages 220-224, XP009070105 ISSN: 0031-6865
- BRUHL P: "PFLANZLICHE ARZNEIMITTEL IN DER UROLOGIE PLANT PRODUCTS IN UROLOGIC THERAPY" THERAPIEWOCHE, KARLSRUHE, DE, vol. 34, no. 6, 1984, pages 787-802, XP009070106 ISSN: 0040-5973
- MALTERUD K.E. ET AL.: 'Flavonoids from orthosiphos spicatus' PLANTA MEDICA vol. 55, no. 6, 1989, pages 569 - 570, XP002975812
- SUMARYONO W. ET AL.: 'Qualitative and quantitative analysis of the phenolic constituents from orthosiphon aristatus' PLANTA MEDICA vol. 57, no. 2, 1991, pages 176 - 480, XP002975813
- LYCKANDER INGE M. ET AL.: 'Lipopholic flavonoids from orthosiphon spicatus as inhibitors of 15-lipoxygenase' ACTA PHARM. NORD vol. 4, no. 3, 1992, pages 159 - 166, XP002975814
- MEIER B. ET AL.: 'Seyo ninjinboku (vitex agnus-castus) to rinsho shiken o moto ni konin sareta shokubutsu chiryoyaku eno michinori' FOOD PROCESSING vol. 36, no. 9, 2001, pages 58 - 61, XP002975815
- EMI OKUYAMA ET AL.: 'Pharmacologically active components of viticis fructus (vitex rotundifolia). I. The components having vascular relaxation effects' NATURAL MEDICINES vol. 52, no. 3, 1998, pages 218 - 225, XP002975816

## Description

The present invention relates to the use of at least one member selected from the group consisting of plants of the genus *Orthosiphon* in the family *Lamiaceae* and extracts thereof for the manufacture of a composition for oral use for stimulating hair growth, which exhibits excellent effects for hair loss prevention, hair regrowth, etc.

Recently, an increasing number of men and women are said to be suffering from thinning hair or hair loss caused by various changes in their social environment, such as an increase in stress factors and changes in eating habits, and thus the social expectations and needs for hair restorers have been increasing. Hitherto, hair restorers containing a variety of active ingredients that are intended to eliminate or alleviate the causes of thinning hair or hair loss have been developed. For example, hair restorers containing swertia herb extract or tocopherol acetate to increase the blood flow to the hair roots, or hinokitiol to improve scalp metabolism have been developed and used to prevent or treat alopecia.

It is believed, however, that various factors, such as hereditary factors, stress, eating habits and aging, are combined in a complicated manner to cause thinning hair or hair loss. Thus, no satisfactory effects of hair loss prevention or hair regrowth can be obtained simply by adding swertia herb extract or other ingredients to improve blood flow or scalp metabolism, like hitherto known hair restorers.

Cat's whiskers (Orthosiphon aristatus (Blume) Miq.) and Orthosiphon stamineus Benth. are perennial plants of the genus Orthosiphon in the family Lamiaceae, and distributed across India, Southeast Asia, the northern part of Australia, Okinawa in Japan, and elsewhere. In India, Malaysia and Indonesia, these plants are used as important folk medicines and known to have effects for alleviating the symptoms of acute or chronic nephritis, cystitis, urolithiasis, cholelithiasis, rheumatic arthritis, diabetes and other diseases. Especially in Indonesia, these plants are called "kumis kuching" and are taken as effective folk medicines against nephritis, urolithiasis, etc.

Japanese Unexamined Patent Publication Nos. 1998-29924, 1999-228337, 2000-95663 and 2001-224330 reported use of plants of the genus Orthosiphon in the family Lamiaceae or extracts thereof as external medicines to be applied to the scalp, such as hair tonics or hair restorers. However, these medicines do not have satisfactory effects.

Use of plants of the genus Orthosiphon in the family Lamiaceae as a hair growth stimulant for oral use has not hitherto been known.

The main object of the present invention is to provide a hair growth stimulant for oral use that has excellent hair growth activity and significant effects for hair loss prevention, hair regrowth promotion, etc.

The present inventors conducted extensive research to achieve the above object, and found that oral administration of a plant of the genus Orthosiphon in the family Lamiaceae or an extract of the plant exhibits a higher hair growth effect than external use thereof. The inventors carried out further research and accomplished the present invention.

The solution to the above technical problem is achieved by providing the subject matter defined in the claims.

The present invention uses the following hair growth stimulants for oral use and food for hair growth.
1. A hair growth stimulant for oral use comprising at least one member selected from the group consisting of plants of the genus Orthosiphon in the family Lamiaceae, and extracts thereof.
2. The hair growth stimulant for oral use according to item 1, wherein the plants of the genus Orthosiphon in the family Lamiaceae are at least one member selected from the group consisting of cat's whiskers (Orthosiphon Aristatus (Blume) Miq.)), Orthosiphon grandiflorus Bold., Orthosiphon rubicundus Benth., Orthosiphon spicatus Benth., and Orthosiphon stamineus Benth.
3. The hair growth stimulant for oral use according to any one of items 1 or 2, which is a food for hair growth.

The present invention is described below in detail.

### Plants of the genus Orthosiphon in the family Lamiaceae

Examples of plants of the genus Orthosiphon in the family Lamiaceae usable in the present invention include cat's whiskers (Orthosiphon aristatus (Blume) Miq.)), Orthosiphon grandiflorus Bold., Orthosiphon rubicundus Benth., Orthosiphon spicatus Benth., Orthosiphon stamineus Benth., etc.

In the present invention, plants of the genus Orthosiphon in the family Lamiaceae may be used as ground products of raw or dried plants, or as extracts obtained by effectively extracting active ingredients from the plants.

The part of the plants to be used is not limited. The aboveground parts, such as stems, leaves and flowers, the subterranean parts, such as roots, or the whole plant can be used, with the aboveground parts being especially preferable.

Extracts of the plants can be obtained by chopping the raw or dried plant and subjecting the chopped plant to extraction with a suitable solvent, which is preferably water, a lower alcohol or a mixture thereof.

Lower alcohols usable for extraction include C₁₋₃ alcohols, such as methanol, ethanol, etc., among which ethanol is most preferable. These alcohols may also be hydrous alcohol (water content: 0.1 to 99.9%).

The extraction can be performed by a batch process, percolation process, reflux process or other known processes. The proportion of the extraction solvent is not limited, and is suitably 2 to 1000 parts relative to 1 part of the chopped raw or dried plant. The extraction can be carried out at room temperature or with heating.

Preferably, the extraction is performed at a temperature range of room temperature to about 80°C for about 1 hour to about 10 hours while stirring under mild conditions. The extraction may also be carried out by adding the solvent dropwise to a cylinder filled with the chopped raw or dried plant.

The part of the plants to be used is not limited, and the roots, trunks, branches, leaves, flowers, fruit and other parts are usable.

Extracts of the plants can be obtained by chopping the raw or dried plant and then subjecting the chopped plant to extraction with a suitable solvent, which is preferably water, a lower alcohol or a mixture thereof.

Lower alcohols usable for extraction include C₁₋₃ alcohols, such as methanol, ethanol, etc., among which ethanol is most preferable. The alcohols may be hydrous alcohol (water content: 0.1 to 99.9%).

The extraction can be performed by a batch process, percolation process, reflux process or other known processes. The amount of the extraction solvent is not limited, and is suitably 2 to 1000 parts relative to 1 part of the chopped raw or dried plant. The extraction can be carried out at room temperature or with heating.

Preferably, the extraction is performed at a temperature range of room temperature to about 80°C for about 1 hour to about 10 hours while stirring under mild conditions. The extraction may be carried out by adding the solvent dropwise to a cylinder filled with the chopped raw or dried plant.

### Hair growth stimulant for oral use

The hair growth stimulant for oral use of the present invention comprises, as an active ingredient, at least one member selected from the group consisting of plants of the genus Orthosiphon in the family Lamiaceae, and extracts thereof.

The hair growth stimulant for oral use of the present invention can be used as a preparation that is suitable as a health food, functional food, supplement or the like. The preparation can also be used as a medicine.

The hair growth stimulant for oral use of the present invention may consist solely of a plant or plants of the genus Orthosiphon in the family Lamiaceae, and/or an extract or extracts thereof, or may be prepared by mixing the plant(s) and/or extract(s) with a material that is used as a carrier and then processing the mixture into a powder, mass, liquid or other form.

Since plants of the genus Orthosiphon in the family Lamiaceae, and extracts thereof, show no toxicity for humans, the amount of the plant or plants and/or an extract or extracts thereof contained in the hair growth stimulant for oral use is not limited, and is preferably such that an adult human can orally ingest 0.1 to 100 g, and preferably 1 to 60 g (calculated as dried plant) of the plant or plants and/or extract or extracts thereof per day, divided into 1 to 3 doses.

The hair growth stimulant for oral use of the present invention may contain suitable additives as required.

The hair growth stimulant for oral use of the present invention can be formulated into an easily usable form, such as tablets, powder, granules, capsules, chewable tablets, liquid, etc.

### Food

The hair growth stimulant for oral use of the present invention can be used in a so-called "food form", such as a food for hair growth.

The food comprising the hair growth stimulant for oral use of the present invention, such as a food for hair growth, can be prepared by processing the hair growth stimulant for oral use alone, or by processing a mixture of the hair growth stimulant for oral use and an arbitrary material usable for food preparation, into a powder, mass, liquid or other form.

The food comprising the hair growth stimulant for oral use of the present invention used for a food can be made into various forms, such as a mass, liquid, syrup, powder, jelly, etc., in a routine manner.

Examples of food forms include soft drinks, juices, teas and other beverages (drink preparations); powdered juices, powdered soups and other powdered food; cookies, biscuits, cereals, chewing gums, candies, gummy candies, tablets, wafers, rice crackers and other confections and snacks; etc.

The food comprising the hair growth stimulant for oral use of the present invention may further contain other ingredients generally used in foods, to such an extent that the effects of the invention are not hindered. Examples of usable ingredients include other medicinal ingredients, nutrients, animal and plant ingredients, excipients, extenders, sweeteners, flavors, colors, preservatives, emulsifiers, solubilizers, polyhydric alcohols and ester derivatives thereof, organic and inorganic acids and salts thereof, water-soluble polymers, etc.

The following Experiment and Examples are intended to illustrate the present invention in further detail, and not to limit the scope thereof.

### Experiment

### (i) Preparation of extract

One hundred grams of a dried whole plant of cat's whiskers, simpleleaf chastetree Reference Examples or lilac chastetree Reference Examples was chopped. Ethanol (2000 ml) was added, and the resulting mixture was heated to 70°C for 2 hours to perform extraction. After filtration, 2000 ml of ethanol was further added to the residue, and extraction was carried out for 2 hours more. The filtrates obtained by the two extraction steps were combined, concentrated under reduced pressure, and freeze-dried to obtain an extract.

### (ii) Test for hair regrowth promotion by external or oral use, and evaluation of the test results

An area of about 2 x 4 cm was shaved on the back of nine 8-week-old C3H/He male mice (average weight: 25 g).

From the day following the day of shaving, 0.1 ml of a 50% aqueous ethanol solution containing 1 wt.% of the extract obtained in (i) was applied to the shaved area of the group for testing external use, once a day for 10 days.

In the group for testing oral use, a 0.5% aqueous carboxymethylcellulose solution containing 5 wt.% of the extract obtained in (i) was orally administered in a dose containing 1 g of the extract per kg of body weight, once a day for 10 days.

Hair regrowth effect was evaluated by comparing the hair restoration in the shaved area. The average of the hair restoration results for the nine mice in each group was used for the comparison.

The hair regrowth promotion effect was expressed as a percentage relative to the hair restoration of the control group (external use of 50% ethanol or oral use of 0.5% carboxymethylcellulose), which was set to 100%, and the increment was presented as a hair regrowth promotion ratio. Table 1 shows the test results of the cat's whiskers extract.

**Table 1**

| | Test substance | Hair restoration ratio (%) | Hair regrowth promotion ratio (%) |
|---|---|---|---|
| External use | Control | 100 | |
| | Cat's whiskers extract | 121 | 21 |
| Oral use | Control | 100 | |
| | Cat's whiskers extract | 185 | 85 |

Table 1 reveals that the oral use of the cat's whiskers extract exhibits a higher hair regrowth promotion effect than the external use thereof. Similar results were obtained with respect to the simpleleaf chastetree extract Reference Examples and lilac chastetree extract Reference Examples.

### Example 1: Formulation of sugar-coated tablets

The extracts prepared in the Experiment and other ingredients were mixed in the proportions shown in Examples 1-A to 1-C, and made into sugar-coated tablets in a routine manner.

### Example 1-A

| Ingredient | Proportion (%) |
|---|---|
| Dolomite | Balance |
| (containing 20% of calcium and 10% of magnesium) | |
| Powdered maltitol syrup | 20 |
| Lactose | 17 |
| Sucrose fatty acid ester | 3 |
| Cat's whiskers extract | 5 |

### Example 1-B (Reference Example)

| Ingredient | Proportion (%) |
|---|---|
| Dolomite | Balance |
| (containing 20% of calcium and 10% of magnesium) | |
| Powdered maltitol syrup | 20 |
| Lactose | 17 |
| Sucrose fatty acid ester | 3 |
| Lilac chastetree extract | 5 |

### Example 1-C (Reference Example)

| Ingredient | Proportion (%) |
|---|---|
| Dolomite | Balance |
| (containing 20% of calcium and 10% of magnesium) | |
| Powdered maltitol syrup | 20 |
| Lactose | 17 |
| Sucrose fatty acid ester | 3 |
| Simpleleaf chastetree extract | 5 |

### Example 2: Formulation of tablets

The extracts prepared in the Experiment and other ingredients were mixed in the proportions shown in Examples 2-A to 2-C, and made into tablets in a routine manner.

### Example 2-A

| Ingredient | Proportion (%) |
|---|---|
| Dextrin | Balance |
| Powdered maltitol syrup | 20 |
| Lactose | 20 |
| Trehalose | 10 |
| Cat's whiskers extract | 5 |
| Aspartame | Trace |
| Flavor | Trace |

### Example 2-B (Reference Example)

| Ingredient | Proportion (%) |
|---|---|
| Dextrin | Balance |
| Powdered maltitol syrup | 20 |
| Lactose | 20 |
| Trehalose | 10 |
| Lilac chastetree extract | 5 |
| Aspartame | Trace |
| Flavor | Trace |

### Example 2-C (Reference Example)

| Ingredient | Amount (%) |
|---|---|
| Dextrin | Balance |
| Powdered maltitol syrup | 20 |
| Lactose | 20 |
| Trehalose | 10 |
| Simpleleaf chastetree extract | 5 |
| Aspartame | Trace |
| Flavor | Trace |

### Example 3: Formulation of drink preparation

The extracts prepared in the Experiment and other ingredients were mixed in the proportions shown in Examples 3-A to 3-C, and made into drink preparations in a routine manner.

### Example 3-A

| Ingredient | Amount (%) |
|---|---|
| Cat's whiskers extract | 5 |
| Sucrose | 2 |
| Ascorbic acid | 1.5 |
| Preservative | Trace |
| Flavor | Trace |
| Purified Water | Balance |

### Example 3-B (Reference Example)

| Ingredient | Proportion (%) |
|---|---|
| Lilac chastetree extract | 5 |
| Sucrose | 2 |
| Ascorbic acid | 1.5 |
| Preservative | Trace |
| Flavor | Trace |
| Purified water | Balance |

### Example 3-C (Reference Example)

| Ingredient | Amount (%) |
|---|---|
| Simpleleaf chastetree extract | 5 |
| Sucrose | 2 |
| Ascorbic acid | 1.5 |
| Preservative | Trace |
| Flavor | Trace |
| Purified water | Balance |

The hair growth stimulant for oral use of the present invention, i.e., a hair growth stimulant for oral use comprising at least one member selected from the group consisting of plants of the genus Orthosiphon in the family Lamiaceae, and extracts thereof, has an excellent hair growth activity and exhibits excellent effects of hair loss prevention, hair regrowth promotion, etc. Further, the food comprising the hair growth stimulant shows excellent effects of hair loss prevention, hair regrowth promotion, etc., and are useful as foods for hair regrowth and like purposes.

## Claims

1. Use of at least one member selected from the group consisting of plants of the genus *Orthosiphon* in the family *Lamiaceae* and extracts thereof for the manufacture of a composition for oral use for stimulating hair growth.

2. Use according to claim 1, wherein the plants of the genus *Orthosiphon* in the family *Lamiaceae* are selected from the group consisting of cat's whiskers (*Orthosiphon aristatus* (Blume) Miq.)), *Orthosiphon grandiflorus* Bold., *Orhosiphon rubicundus* Benth., *Orthosiphon spicatus* Benth., and *Orthosiphon stamineus* Benth.

3. The use according to claim 1, wherein said composition for oral use comprises 0.1 to 100 g of the plant calculated as a dry weight or extract thereof.

4. The use according to claim 1, wherein said extract is obtained by extracting with a solvent selected from the group consisting of water, C₁₋₃ alcohols and a mixture thereof.

5. The use according to claim 1, wherein said composition for oral use is food or medicine.

6. The use according to claim 1, wherein said composition for oral use is in a form selected from the group consisting of tablets, powder, granules, capsules, chewable tablets and liquid.

7. The use according to claim 5, wherein said food is in a form selected from the group consisting of a mass, liquid, syrup, powder and jelly.

## Patentansprüche

1. Verwendung von zumindest einem Mitglied, ausgewählt aus der Gruppe, bestehend aus Pflanzen der Gattung *Orthosiphon* in der Familie *Lamiaceae* und Extrakten davon, für die Herstellung einer Zusammensetzung zur oralen Anwendung zum Stimulieren des Haarwachstums.

2. Verwendung nach Anspruch 1, wobei die Pflanzen der Gattung *Orthosiphon* in der Familie *Lamiaceae* aus der Gruppe, bestehend aus Katzenbart (*Orthosiphon aristatus* (Blume) Miq.), *Orthosiphon grandiflorus* Bold., *Orthosiphon rubicundus* Benth., *Orthosiphon spicatus* Benth. und *Orthosiphon stamineus* Benth., ausgewählt sind.

3. Verwendung nach Anspruch 1, wobei die Zusammensetzung zur oralen Anwendung 0,1 bis 100 g der Pflanze, berechnet als Trockengewicht oder Extrakt davon, umfaßt.

4. Verwendung nach Anspruch 1, wobei der Extrakt durch Extrahieren mit einem Lösungsmittel, ausgewählt aus der Gruppe, bestehend aus Wasser, C₁₋₃ Alkoholen und Mischungen davon, erhalten wird.

5. Verwendung nach Anspruch 1, wobei die Zusammensetzung zur oralen Verabreichung ein Lebensmittel oder ein Arzneimittel ist.

6. Verwendung nach Anspruch 1, wobei die Zusammensetzung zur oralen Verabreichung in einer Form, ausgewählt aus der Gruppe, bestehend aus Tabletten, Pulver, Granulat, Kapseln, Kautabletten und Flüssigkeit, vorliegt.

7. Verwendung nach Anspruch 5, wobei das Lebensmittel in einer Form, ausgewählt aus der Gruppe, bestehend aus einer Masse, Flüssigkeit, Sirup, Pulver und Gelee, vorliegt.

## Revendications

1. Utilisation d'au moins un élément choisi dans le groupe constitué par les plantes du genre *Orthosiphon* de la famille des Lamiacées et des extraits de celles-ci pour la fabrication d'une composition destinée à une utilisation par voie orale pour stimuler la croissance capillaire.

2. Utilisation selon la revendication 1, dans laquelle les plantes du genre *Orthosiphon* dans la famille des Lamiacées sont choisies dans le groupe constitué par la moustache de chats (*Orthosiphon aristatus* (Blume) Miq.), *Orthosiphon grandiflorus* Bold., *Orthosiphon rubicundus* Benth., *Orthosiphon spicatus* Benth., et *Orthosiphon stamineus* Benth.

3. Utilisation selon la revendication 1, dans laquelle ladite composition destinée à une utilisation par voie orale comprend de 0,1 à 100 g de plante calculé comme le poids sec ou l'extrait de celle-ci.

4. Utilisation selon la revendication 1, dans laquelle ledit extrait est obtenu par extraction avec un solvant choisi dans le groupe constitué par l'eau, les alcools en C₁₋₃, et un mélange de ceux-ci.

5. Utilisation selon la revendication 1, dans laquelle ladite composition destinée à une utilisation par voie orale est un aliment ou un médicament.

6. Utilisation selon la revendication 1, dans laquelle ladite composition destinée à une utilisation par voie orale se trouve sous une forme choisie dans le groupe constitué par les comprimés, une poudre, les granules, les gélules, les comprimés à mâcher et un liquide.

7. Utilisation selon la revendication 5, dans laquelle ledit aliment se trouve sous une forme choisie dans le groupe constitué par une pâte, un liquide, un sirop, une poudre et une gelée.
